# EUROPEAN PATENT APPLICATION

(11) **EP 1 298 219 A1**
(43) Date of publication of application: **02.04.2003**
(21) Application number: 01945806.6
(22) Date of filing: 03.07.2001
(51) Int. Cl.: C12Q 1/02, C07K 16/00, G01N 33/563, A61K 39/395

(54) **METHOD OF ASSAYING THE FUNCTION OF FC FRAGMENT OF ANTIBODY**

(30) Priority: 04.07.2000 JP 2000202622
(71) Applicant: CHUGAI SEIYAKU KABUSHIKI KAISHA, Tokyo, 115-8543 (JP)
(72) Inventor: SUGO, Izumi, C/O CHUGAI SEIYAKU KABUSHIKI KAISHA, Gotemba-shi, Shizuoka 412-8513 (JP)
(74) Representative: Maschio, Antonio
(86) International application number: JP0105772
(87) International publication number: WO02002801

(57) **Abstract**

A method for measuring the function of Fc moiety of an antibody, said method comprising the steps of:
(1) immobilizing a test antibody;
(2) culturing a Fc receptor-expressing cell line in the presence of the above immobilized antibody; and
(3) measuring the reaction in the cell resulting from the function of Fc receptor of said Fc receptor-expressing cell.

## Description

### Field of Invention

The present invention relates to a novel method for measuring the function of Fc moiety of an antibody.

### Background Art

Antibody-dependent cell-mediated cytotoxicity (ADCC) is a phenomenon which is exhibited by the antigen-binding ability of Fab moiety of an antibody and the immune cell-activating ability of Fc moiety of an antibody. The ADCC activity is one of the major action mechanisms of antibody drugs such as the anti-cancer effect and the anti-viral effect, and methods of measuring the ADCC activity have been widely used for the demonstration of pharmacological effects etc. However, the methods of measuring the ADCC activity are intended to measure the activity of an antibody as a whole, and cannot separately measure the antigen-binding ability of the Fab moiety and the immune cell-activating ability of the Fc moiety.

Thus, when there is a decrease in the ADCC activity of an antibody, methods of measuring the ADCC activity cannot specify whether it was caused by a change in the antigen-binding ability of the Fab moiety or by a change in the Fc receptor-binding ability of the Fc moiety. For example, even a change in the structure of the Fab moiety, and thereby a change in antigen-binding ability, may cause a decrease in the ADCC activity, or even a change in the structure of the Fc moiety, and thereby a change in the Fc receptor-binding ability, may cause a decrease in the ADCC activity.

For the antigen-binding ability of the Fab moiety, ELISA, BIOACORE etc. can confirm the specificity of antigen binding and the activity of binding to antigen, but no method has been established that can measure the Fc receptor-binding ability of the Fc moiety. Therefore, when the long-term storage of an antibody caused a decrease in the ADCC activity, it was conventionally impossible to clearly determine whether it was caused by a structural change in the Fab moiety or a structural change in the Fc moiety.

On the other hand, as a method for confirming quantitatively the cell activation of the Fc moiety of an antibody via the Fc receptor, there is known a method that measures a change in IL-8 production induced by the binding of the Fc receptor to the Fc moiety of an antibody using peripheral blood mononuclear cells (J. Immunol., 155:3161-3167 (1995)). However, the method of using peripheral blood mononuclear cells has problems in terms of simplicity, reproducibility and quantitative nature of the method, and there has been a need for a method that permits the simple, reproducible and quantitative measurement of the activity of the Fc moiety using a cell line. Furthermore, it has not been demonstrated that the Fc receptor-binding ability of Fc moiety of an antibody actually correlates with the ADCC activity, and a simple method has not been established that can confirm changes in the ADCC activity associated with a structural change in the Fc moiety.

### Disclosure of the Invention

Thus, it is an object of the present invention to provide a method that permits the measurement of the activity of the Fc moiety per se separately from the activity of the Fab moiety. Such a method of assessing the ADCC activity based on the activity of the Fc moiety could be utilized in the understanding of the bulk of a pharmaceutical containing an antibody as an active ingredient, in the management of homogeneity of the bulk and of production control of the bulk, and the like.

After intensive and extensive research to solve the above problems, the present inventors have found that the activity of Fc can be measured by culturing cells that express Fc receptor in the presence of an antibody having the Fc moiety which is a test sample etc., and, then, by measuring the reaction in the cells resulting from the function of the Fc receptor, and thereby have completed the present invention.

Thus, it is an object of the present invention to provide a method for measuring the function of Fc moiety of an antibody, said method comprising the steps of:
(1) immobilizing a test antibody;
(2) culturing a Fc receptor-expressing cell line in the presence of the above immobilized antibody; and
(3) measuring the reaction in the cell resulting from the function of Fc receptor of said Fc receptor-expressing cell.

Preferably, said Fc receptor-expressing cell is a cell line that has been pretreated with a cytokine.

More preferably, the cytokine with which said Fc receptor-expressing cell is pretreated is interferon γ.

Preferably, the pretreatment with said interferon γ comprises culturing the Fc receptor-expressing cell in the presence of 1-10000 U/ml IFN-γ for 12-96 hours.

Preferably, said cell line is the THP-1 cell.

Preferably, the reaction by said cell is cytokine production.

More preferably, the cytokine production is the production of IL-8.

Preferably, the immobilization of said test antibody is immobilization onto a plate.

Preferably, said test antibody is an antibody having the constant region derived from a human antibody.

More preferably, said test antibody is an antibody having the Fc moiety derived from a human.

Preferably, the antibody having the constant region derived from said human antibody is a chimeric antibody, a humanized antibody or a human antibody.

More preferably, said humanized antibody is anti-HM1.24 antibody.

The present invention also provides a method of quality control of antibody by confirming the absence of changes in the function or the structure of Fc moiety of the antibody by using the above method.

The present invention also provides an antibody that is produced by said quality control method.

The present invention also provides a pharmaceutical composition comprising said antibody as an active ingredient.

### Brief Description of Drawings

Fig. 1 is a drawing which shows the result of flow cytometry in which FcγRI(CD64) and FcγRIII(CD16) expressed on the cells of the THP-1 cell line were detected with mouse anti-human CD16 antibody and mouse anti-human CD64 antibody, respectively.
Fig. 2 is a graph which shows the amount of IL-8 produced when the THP-1 cells activated with IFN-γ were cultured in the presence of the immobilized humanized HM1.24 antibody.
Fig. 3 is a graph which shows that there are no differences in the amount of antibody immobilized between an active humanized antibody HM1.24 and a deteriorated version of humanized antibody HM1.24.

### Embodiments for Carrying Out the Invention

The antibody which is a subject of the method of the present invention may be, but is not limited to, anti-HM1.24 antibody and various other antibodies having an ADCC activity such as anti-HER2 antibody (WO 89/06692), the form of which antibody may be any form as long as it contains the Fc moiety, and can be applied to a polyclonal antibody, a monoclonal antibody, a chimeric antibody, a humanized antibody and the like.

The constant region of antibody refers to a region other than the variable region proposed by Kabat et al. (Kabat, "Sequence of Proteins of Immunological Interest," US Department of Health and Human Services (1983)). The Fc moiety refers to a region which is not involved in the binding with the antigen and which is primarily responsible for the effector function among the fragments cleaved with a proteolytic enzyme, papain.

The test antibody may be any antibody as long as it can bring the Fc moiety receptors of the cells into the proximity of one another and, as one such method, antibody can be immobilized. The method of immobilization is not limited as long as it permits the immobilization of antibody onto a carrier having no cell-stimulating effect. For example, as mentioned in Examples, antibody may be immobilized by coating it on the bottom of a plate for measurement. Furthermore, it is also possible to immobilize antibody on beads, with which the cells may be cultured.

For example, a given amount of beads having no cell-stimulating effect such as the Microbeads Basic (Daiichi Kagaku Yakuhin) is added to an antibody solution of a known concentration to immobilize nonspecifically the antibody onto the beads. A given amount of such beads is added to the cell solution to stimulate the cells, and the resulting reaction in the cells, for example the production of cytokines such as IL-8, may be examined. A thin film may also be used for immobilization. For example, a given amount of antibody solution of a known concentration is placed on a PVP free polycarbonate filter (Ieda Kagaku: NEURO PROBE PVP free) and dried as appropriate. This filter is added into a cell suspension and is reacted for a given time to stimulate the cells, and the resulting reaction in the cells, for example the production of cytokines such as IL-8, may be examined.

As the cell line to be used in the measurement, any cell line can be used for the present measurement system as long as it is Fc receptor-expressing cell and it can induce the production of cytokines in response to the stimulation of the Fc receptor. For example, when the test antibody is an antibody having the constant region of a human antibody such as a human antibody, a humanized antibody and a chimeric antibody, the cells used in the measurement include a human cell line, preferably a monocytic cell line. Specifically, there can be mentioned THP-1, U937 and HL60 (Molecular Immunology 21, 1984; Molecular Immunology 21, 1992). Besides, a NK-like cell line such as NK3.3, VL-186-1 and LAK (lymphokine activated killer) can also be used.

In order to enhance the reactivity of Fc receptor of cells to stimulation, as described in Examples, pretreatment may be effected with a cytokine such as interferon γ. Cytokines that can be used for the pretreatment of such cells include IL-2, IL-12, IL-6, IFN-α (Hybridoma 18:63-68, 1999; EP 0302435) and IL-10 (J. Immunology 149:4048-4052, 1992), in addition to interferon γ.

Pretreatment with such cytokines may be effected for about 12-96 hours, preferably about 24-72 hours, preferably 40-48 hours.

As cytokines produced in response to stimulation via the Fc receptor, there can be mentioned TNF-α (J. Leukocyte Biology 64:124-133, 1998), MCP-1 (BBRC 159:249-255, 1989) and IL-6 (J. Immunology 145:1337-1342, 1990) in addition to IL-8. The concentration of these cytokines in the culture liquid may be measured using a commercially available ELISA kit, and the amount of a cytokine produced may be measured using a reverse phase HPLC, a chemotaxis assay (bioassay), a quantitative RP-PCR, Northern blotting or Western blotting.

Stimulation via the Fc receptor can also be detected by measuring an increase in the level of intracellular calcium using a calcium sensor reagent in addition to cytokines. Furthermore, when the cells are NK-like cells, Perforin, Granzyme and NO can be measured.

### Examples

The present invention will now be explained with reference to the following Examples.

### Example 1. Measurement of the ability of producing IL-8 from the THP-1 cells by stimulation via Fc

Humanized anti-HM1.24 antibody was prepared by the method described in International Patent Publication WO 99/18212. Soluble HM1.24 antigen was also prepared by the method described in International Patent Publication WO 99/00885. Humanized anti-HM1.24 antibody 99A01, a standard, was purified and then stored at 4°C prior to use. The deteriorated versions, C6-60-2W and C7-60-2W, of humanized anti-HM1.24 antibody used were stored in a citrate buffer, pH 6.0, at 60°C for two weeks and in a citrate buffer, pH 7.0, at 60°C for two weeks, respectively.

Incidentally, HM1.24 antigen is a tumor antigen produced by a human multiple myeloma cell line, and Escherichia coli (E. coli) containing a plasmid pRS38-pUC19 in which a gene encoding the soluble HM1.24 antigen has been inserted has been internationally deposited under the provisions of the Budapest Treaty on October 5, 1993, with the National Institute of Bioscience and Human Technology, Agency of Industrial Science and Technology, of 1-3, Higashi 1-chome, Tsukuba city, Ibalaki pref., Japan, as the accession number FERM BP-4434.

E. coli DH5α (pUC19-RVLa-AHM-gκ) and E. coli DH5α (pUC19-RVH-gγ1) containing a plasmid pUC19-RVLa-AHM-gκ and a plasmid pUC19-RVHr-AHM-gγ1, respectively, having a gene encoding the humanized L chain of antibody to the HM1.24 antigen have been internationally deposited on August 29, 1996 with the above depository as FERM BP-5645 and FERM BP-5643, respectively. Furthermore, E. coli DH5a (pUC19-RVHs-AHM-gγ1) containing a plasmid pUC19-RVHs-AHM-gγ1 having a gene encoding the humanized H chain of antibody against HM1.24 antigen has been internationally deposited on September 29, 1997 with the above depository as FERM BP-6127. Thus, by expressing these plasmids in animal cells, humanized anti-HM1.24 antibody can be obtained.

THP-1 cells are a monocytic cell line and are known to express FcγRI(CD64) and FCγRIII(CD16) thereon (Schlossman S.F. et al., Leukocyte typing V. Oxford University Press, 1995). Accordingly, Fc receptor expressed on the THP-1 cells was confirmed by the flow cytometric method. 1 x 10⁶ THP-1 cells (ATCC; 202-T1B, Batch F-11793), after washing three times with D-PBS (Nissui; Code 05913, Dulbecco's PBS "Nissui"), were reacted with 10 µl of mouse anti-human CD16 antibody (Serotec; No. MCA1193XZ, Batch 1197: 1 mg/ml) or mouse anti-human CD64 antibody (Ancell; No. 216-020, Lot 9607013: 1 mg/ml) at 4°C for four hours.

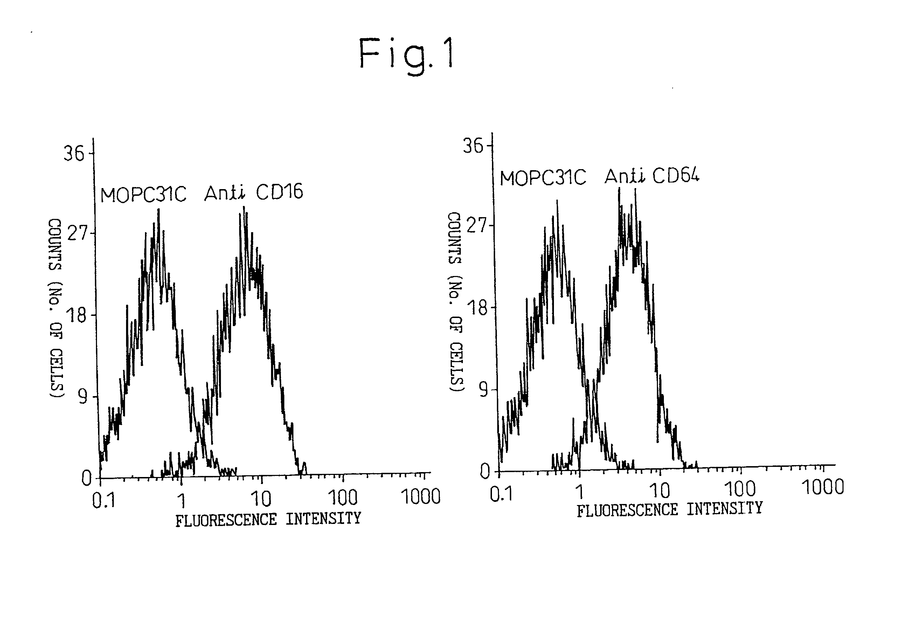

After washing three times with D-PBS, the cells were reacted with 4 µl of FITC-goat anti-mouse IgG antibody (Becton Dickinson; No. 349031, Lot 60101: 1 mg/ml) at 4°C for 1 hour. After washing three times with D-BPS, the cells were suspended in 1 ml of D-PBS and analyzed using a flow cytometer. In stead of anti-CD antibody as a control, 10 µl of MOPC31c (1 mg/ml) was reacted with the THP-1 cells. As a result, the expression of Fc receptors, CD16(Fc γRI) and CD64(Fc γRIII) was confirmed (Fig. 1).

Since the THP-1 cells were confirmed to be expressing Fc receptor, it was investigated whether the effect of producing IL-8 can be confirmed by the stimulation of the immobilized humanized anti-HM1.24 antibody. Humanized anti-HM1.24 antibody (99A01: 21.77 mg/ml) was serially diluted with D-PBS, and immobilized onto a 96-well plate (100 µl/well, 4°C: overnight). The THP-1 cells activated with IFN-γ were plated on a 96-well plate at 100 µl/well and cultured for about 18 hours, and the amount of IL-8 in the supernatant was measured. The IFN-γ-activated THP-1 cells were prepared by culturing for 48 hours in the presence of 100 U/ml (10 ng/ml) IFN-γ.

After culturing with IFN-γ, the THP-1 cells were washed with RPMI1640 medium containing 10% FCS, and adjusted to 3 x 10⁶ cells/ml with the same medium, which was then plated on an antibody-immobilized 96-well plate at 100 µl/well, and cultured for about 18 hours. The cells after culturing were centrifuged (1500 rpm, 15 min), the culture supernatant was recovered at aliquots of 80 µl, and the amount of IL-8 was measured by the IL-8 ELISA kit (Genzyme; DUOSET human IL-8). ELISA measurement was made in duplicates.

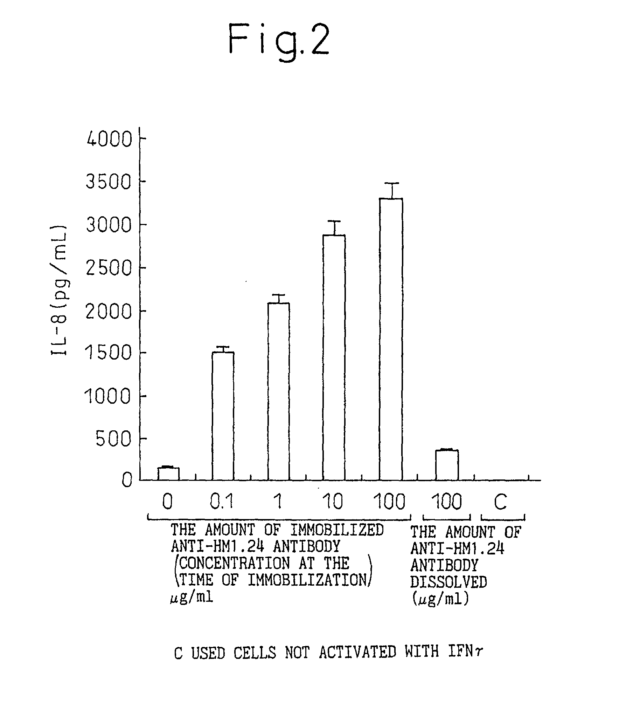

As a control, the THP-1 cells not activated with IFN-γ were similarly plated on wells on which 100 µg/ml of humanized anti-HM1.24 antibody had been immobilized, and the amount of IL-8 produced was measured. Furthermore, without immobilizing humanized anti-HM1.24 antibody, the THP-1 cells were suspended in a medium containing 100 µg/ml of humanized anti-HM1.24 antibody, and the amount of IL-8 produced was similarly measured. As a result, the amount of IL-8 produced by the THP-1 cells increased depending on the amount of immobilized humanized anti-HM1.24 antibody. Furthermore, when humanized anti-HM1.24 antibody was not immobilized and when the THP-1 cells were not activated with IFN-γ, very little production of IL-8 by the THP-1 cells was detected (Fig. 2).

Since it was confirmed that the immobilized humanized anti-HM1.24 antibody stimulates the THP-1 cells resulting in the production of IL-8, the ability of deteriorated preparations of humanized anti-HM1.24 antibody to produce IL-8 was compared. Humanized anti-HM1.24 antibody (99A01: 21.77 mg/ml) and the deteriorated preparations of humanized anti-HM1.24 antibody (citrate buffer, pH 6.0, 60°C, two weeks: 24.01 mg/ml (C6-60-2W); citrate buffer, pH 7.0, 60°C, two weeks: 24.61 mg/ml (C7-60-2W)) were serially diluted with D-PBS, and then were immobilized on two 96-well plates at five wells for each concentration (100 µl/well: 4°C, overnight).

To one of the two prepared plates, the THP-1 cells were plated, and the amount of IL-8 produced was measured. The other plate was used for confirmation of the amount of antibody immobilized. Thus, after blocking with D-PBS containing 1% BSA, alkaline phosphatase (ALP)-labeled anti-human IgGγ antibody (BIOSOURCE; No. AH10305, Lot 6701) was reacted, and the alkaline phosphatase activity after washing was measured using an alkaline phosphatase substrate (Sigma; Sigama 104) to compare the amount of immobilized IgG.

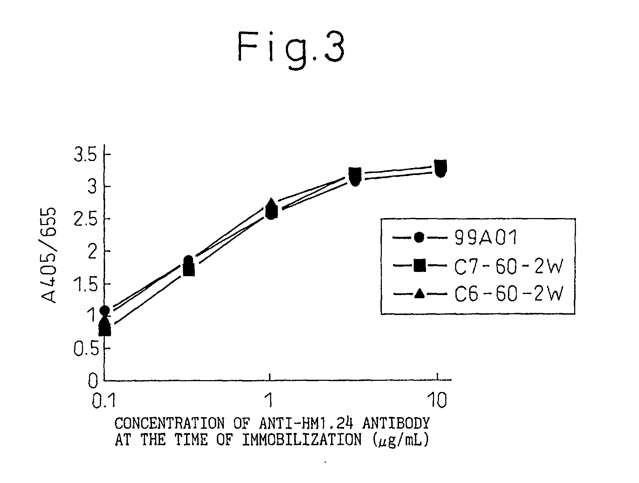

As a result, as shown in Table 1, the effect of promoting IL-8 production by each antibody was 99A01 > C7-60-2W > C6-60-2W. There were no differences noted in the immobilized amount among the antibodies used (99A01, C7-60-2W, C6-60-2W) (Fig. 3).

**Table 1**

| | | | | | | |
|---|---|---|---|---|---|---|
| The amount of IL-8 produced (pg/ml) | | | | | | |

| Humanized anti-HM1.24 antibody (µg/ml) n=5 | 99A01 | | C6-60-2W | | C7-60-2W | |
|---|---|---|---|---|---|---|
| | Mean | Standard deviation | Mean | Standard deviation | Mean | Standard deviation |
| 0 | 713 | 16 | | | | |
| 1 | 6001 | 261 | 4029 | 189 | 5152 | 323 |
| 3 | 14106 | 1029 | 8050 | 347 | 9699 | 555 |

### Example 2. Measurement of antigen-binding ability

As the reduction in function was observed for the immune cell-activating ability of the Fc moiety in the deteriorated versions of humanized anti-HM1.24 antibody, the antigen-binding ability of the Fab moiety was examined using the BIACORE method (BIACORE 1000: BIACORE). As a running buffer, HBS-EP buffer was used at a flow rate of 5 µl/min. Protein was diluted with the HBS-EP buffer unless otherwise specified. Other common procedures for BIACORE were as described in the instruction manual attached.

Using a soluble HM1.24 antigen (370 µg/ml), the immobilization of the soluble HM1.24 antigen onto a sensor chip was carried out by the amine coupling method. One hundred µl of 0.05 mol/l NHS/0.2 mol/l EDC was injected to the Sensor Chip CM5, and the carboxyl groups of carboxymethyl dextran on the sensor chip were activated. Then, 50 µl of 50 µl/ml sHM antigen (the solvent was 10 mmol/l sodium acetate, pH 4.0 or pH 3.2) was injected for immobilization.

Then, 100 µl of 1.0 mol/l ethanolamine HCl (pH 8.5) and 10 µl of Gly-HCl (pH 2.5) were injected to block the excess of active groups. Finally, 5 µl of 10 mmol/l HCl was injected to wash away the non-covalently bound materials.

The soluble HM1.24 antigen-immobilized sensor chip was set, and 10 µl each of 0, 1.25, 2.5, 5 and 10 µl/ml humanized anti-HM1.24 antibody was injected thereinto. The value of Resonance Unit (RU) immediately before (10 seconds before) injection was set as the baseline, and the RU value after the injection (30 seconds after the completion of injection) was measured, and a change in the RU values before and after the injection was set as the bound amount of humanized anti-HM1.24 antibody. The sensor chip was reconstituted with 5 µl of 20 mmol/l HCl.

The concentration of humanized anti-HM1.24 antibody before dilution was calculated from the peak area that was recognized as the monomer fraction by GPC. Humanized anti-HM1.24 antibody (99A01: 21.77 mg/ml) and the deteriorated versions (C6-60-2W, C7-60-2W) of humanized anti-HM1.24 antibody were diluted with the HBS-EP buffer, and the binding ratio of the deteriorated versions of humanized anti-HM1.24 antibody when the standard curve was established with 99A01 was calculated.

As a result, as shown in Table 2, the binding ratio of C6-60-2W was about 76% on the average and that of C7-60-2W was about 58% on the average. Thus, as a result of measuring antigen-binding ability by the BIACORE method, the activity of Fab was confirmed to decrease in the order of 99A01 > C6-60-2W > C7-60-2W.

**Table 2**

| | | | |
|---|---|---|---|
| Quantitation by BIACORE (binding ratio) | | | |

| Humanized anti-HM1.24 antibody (µg/ml) n=3 | 99A01 Mean | C6-60-2W Mean | C7-60-2W Mean |
|---|---|---|---|
| 2.5 | 2.49 (99.4) | 1.99 (79.6) | 1.55 (62.1) |
| 5 | 5.16 (103.2) | 3.64 (72.8) | 2.69 (53.8) |
| Mean binding ratio | 101.3 | 76.2 | 58.0 |

### Example 3. Measurement of ADCC activity

The measurement of ADCC activity was made according to a known method (Current Protocols in Immunology, Chapter 7. Immunologic Studies in Humans, John Wiley & Sons, Inc., 1993).

The effector cells used were mononuclear cells isolated by gravity centrifugation from the peripheral blood of normal healthy humans. Thus, to the peripheral blood of normal healthy humans, an equal amount of PBS was added, and layered on Ficoll-Paque PLUS (Pharmacia), and then centrifuged at 500 g for 30 minutes. The mononuclear cell layer was collected, and after washing three times with RPMI1640 medium (GIBCO) containing 10% FBS (GIBCO), the cell count was adjusted to 5 x 10⁶ /ml with the same culture medium.

The target cells were radiolabeled by culturing 1 x 10⁶ KPMM2 cells together with ⁵¹Cr-sodium chromate (ICN) in RPMI1640 medium containing 10% FBS (GIBCO) at 37°C for 60 minutes, and the cells were then washed three times with the same culture medium to adjust to 2 x 10⁵ /ml. To a U-bottomed 96-well plate (Becton Dickinson), 0.05 ml of 2 x 10⁵ /ml radiolabeled target cells and 0.05 ml of each concentration of two-fold serially diluted anti-HM1.24 antibody were added, and reacted at 4°C for 15 minutes.

Then 0.1 ml of the effector cells was added thereto, which was cultured at 37°C for 4 hours. At this time, the ratio (E:T) of the effector cells (E) to the target cells (T) was set at 35:1. After culturing, 0.1 ml of the culture supernatant was harvested, and radioactivity liberated in the culture supernatant was counted using a gamma counter (ARC-3000: Aloka). For measurement of the maximum radioactivity, 1% NP-40 was used.

Cytotoxicity (%) was calculated based on (A-C)/(B-C) x 100, wherein A represents a radioactivity (cpm) liberated in the presence of antibody, B represents a radioactivity (cpm) liberated by NP-40, and C represents a radioactivity (cpm) liberated by the culture medium alone without antibody. The result is shown in Table 3. ADCC activity was 99A01 > C7-60-2W > C6-60-2W, and correlated with the IL-8 production ability via Fc. These results indicated that the ADCC activity of antibody correlates with the amount produced of IL-8.

**Table 3**

| | | | | | | |
|---|---|---|---|---|---|---|
| % ⁵¹Cr release | | | | | | |

| Humanized anti-HM1.24 antibody (µg/ml) n=5 | 99A01 | | C6-60-2W | | C7-60-2W | |
|---|---|---|---|---|---|---|
| | Mean | Standard deviation | Mean | Standard deviation | Mean | Standard deviation |
| 0.064 | 36.7 | 3.3 | 6.8 | 1.4 | 8.8 | 2.5 |
| 0.128 | 41.2 | 2.5 | 9.7 | 1.7 | 17.3 | 1.4 |
| 0.256 | 43.4 | 1.7 | 12.5 | 1.3 | 20.8 | 1.4 |
| 0.512 | 48.4 | 2.2 | 13.0 | 0.2 | 21.5 | 1.9 |

From the above results, IL-8 production was measured using the THP-1 cells pretreated with IFN-γ, and it was confirmed that IL-8 can be produced at an amount sufficient to be measured depending on the amount of the immobilized antibody.

Furthermore, when the activity of the Fc moiety of the deteriorated versions of humanized anti-HM1.24 antibody was compared with the ADCC activity, the deteriorated versions of humanized anti-HM1.24 antibody having a decreased ADCC activity also had a decreased activity of the Fc moiety in a similar manner to the ADCC activity. The result of comparison of the amount immobilized also indicated that there are no differences in the amount immobilized between the antibodies (99A01, C7-60-2W, C6-60-2W), and that the above difference in the ability of producing IL-8 was not merely due to the difference in the amount immobilized of antibody, but to the difference in the activity derived from the Fc moiety of antibody. On the other hand, the antigen-binding ability using BIACORE exhibited a rank correlation different from ADCC activity on the deteriorated versions.

These results indicated that the method of immobilizing antibody and measuring the amount of IL-8 produced from the THP-1 cells is a method that permits the comparison of immune cell-activating ability by the Fc moiety of antibody, and thus the present method was demonstrated to be a favorable assessment method that measures changes in the structure and function of the Fc moiety resulting from storage of antibody.

## Claims

1. A method for measuring the function of Fc moiety of an antibody, said method comprising the steps of:
(1) immobilizing a test antibody;
(2) culturing a Fc receptor-expressing cell line in the presence of the above immobilized antibody; and
(3) measuring the reaction in the cell resulting from the function of Fc receptor of said Fc receptor-expressing cell.

2. A method according to claim 1 wherein said Fc receptor-expressing cell is a cell line that has been pretreated with a cytokine.

3. A method according to claim 2 wherein the cytokine with which said Fc receptor-expressing cell is pretreated is interferon γ.

4. A method according to claim 3 wherein the pretreatment with said interferon γ comprises culturing the Fc receptor-expressing cell in the presence of 1-10000 U/ml IFN-γ for 12-96 hours.

5. A method according to claim 1 or 2 wherein said cell line is the THP-1 cell.

6. A method according to claim 1 wherein the reaction by said cell is cytokine production.

7. A method according to claim 6 wherein the cytokine production is the production of IL8.

8. A method according to claim 1 wherein the immobilization of said test antibody is immobilization onto a plate.

9. A method according to claim 1 wherein said test antibody is an antibody having the constant region derived from a human antibody.

10. A method according to claim 1 wherein said test antibody is an antibody having the Fc moiety derived from humans.

11. A method according to claim 9 wherein the antibody having the constant region derived from said human antibody is a chimeric antibody, a humanized antibody or a human antibody.

12. A method according to claim 11 wherein said humanized antibody is anti-HM1.24 antibody.

13. A method of quality control of antibody by confirming the absence of changes in the function or the structure of the Fc moiety of the antibody by using the method according to claim 1.

14. An antibody that was produced by said quality control method according to claim 13.

15. A pharmaceutical composition comprising an antibody according to claim 14 as an active ingredient.
